# FASCICULE DE BREVET EUROPEEN

(11) **EP 0 698 392 B1**
(45) Date de publication et mention de la délivrance du brevet: **29.11.2000**
(21) Numéro de dépôt: 95401696.0
(22) Date de dépôt: 17.07.1995
(51) Int. Cl.: A61K 31/59, A61K 31/38, A61K 31/195, A61K 31/19, A61K 31/20

(54) **Compositions contenant et ligand spécifique de RAR alpha et au moins un acide gras**
Zusammensetzungen, welche ein RAR alpha spezifischer Liganden und mindestens eine Fettsäure enthalten
Compositions containing a RAR alpha specific ligand and at least one fatty acid

(30) Priorité: 02.08.1994 FR 9409584
(43) Date de publication de la demande: 28.02.1996
(73) Titulaire: CENTRE INTERNATIONAL DE RECHERCHES DERMATOLOGIQUES GALDERMA (C.I.R.D. GALDERMA), 06560 Valbonne (FR)
(72) Inventeur: Ailhaud, Gérard, F-06100 Nice (FR); Grimaldi, Paul, F-06100 Nice (FR); Safonova, Irima, F-06000 Nice (FR); Shroot, Braham, F-06600 Antibes (FR); Reichert, Uwe, F-06620 Pont du Loup (FR)
(74) Mandataire: Tezier Herman, Béatrice

(56) Documents cités:
- WO-A-93/03713
- PROC ANNU MEET AM ASSOC CANCER RES;33:A565-6 15-07-1992, SPORN MB ET AL 'MOLECULAR AND CELLULAR BASIS FOR THE USE OF RETINOIDS IN CHEMOPREVENTION (MEETING ABSTRACT)'
- NEW ENGL. J. MED.,15-07-1993, 329/3 (177-189), USA, WARRELL R.P. JR. ET AL 'Acute promyelocytic leukemia'

## Description

La présente invention concerne, de manière générale, un procédé et des compositions pour un usage en médecine humaine ou vétérinaire plus particulièrement destinés à accélerer *in vitro* et/ou *in vivo* la différenciation des cellules préadipocytaires en cellules adipocytaires, et ceci dans le but notamment de corriger un éventuel état d'insulino-résistance existant au sein d'un organisme vivant et de traiter et/ou prévenir ainsi, chez cet organisme, les différentes physiopathologies qui peuvent être associées à un tel état.

### Généralités et art antérieur

On sait que certaines cellules de la peau, appelées adipocytes, contiennent des quantités variables de graisses sous la forme de triglycérides, ces triglycérides étant synthétisés *in vivo* à l'intérieur même des adipocytes, selon des réactions de type enzymatique (lipogénèse), à partir des acides gras libres et du glucose (après dégradation de ce dernier en glycérophosphate) circulant dans l'organisme et apportés à celui-ci par l'intermédiaire de certains aliments. Parallèlement, les triglycérides ainsi formés, puis stockés, dans les cellules adipocytes peuvent également se redécomposer, toujours sous l'action d'enzymes spécifiques (lipolyse) contenues dans ces mêmes cellules, en libérant cette fois des acides gras d'une part et du glycérol et/ou des mono- et/ou des di-esters du glycérol d'autre part. Les acides gras ainsi relargués peuvent alors soit diffuser dans l'organisme pour y être consommés ou transformés de différentes façons, soit être recaptés (aussitôt ou un peu plus tard) par les adipocytes pour générer à nouveau des triglycérides par lipogénèse.

Les adipocytes présents dans le tissu adipeux proviennent eux-mêmes, de manière connue, de la transformation de cellules précurseurs originelles, appelées préadipocytes, qui sont des cellules d'apparence fibroplastique capables de se multiplier et de se différencier en adipocytes sous l'action de certains agents hormonaux, comme par exemple l'insuline ou l'hormone de croissance. Chez l'homme, la quantité de préadipocytes isolables du tissu adipeux et susceptibles de se différencier ultérieurement en adipocytes, est inversement proportionnelle à l'âge des individus cependant, mêmes chez les individus agés, il subsiste toujours une proportion de cellules dites "dormantes" susceptibles d'être un jour réactivées. En d'autres termes, tout au long de sa vie, le tissu adipeux contient des préadipocytes capables de se différencier en adipocytes sous l'influence de divers stimuli appropriés. A cet égard, il est déja connu que les acides gras libres d'une part ainsi que, d'autre part, les rétinoïdes naturels, lorsque ces derniers sont utilisés à des concentrations physiologiques (1-10 nM), et les rétinoïdes synthétiques lorsqu'ils sont utilisés à de très faibles concentrations (1 pM - 1 nM), constituent, pris isolément, de bons stimulateurs de la différenciation préadipocytaire.

On sait par ailleurs que l'insuline (qui est une hormone protéique naturelle produite en quantités variables par certaines cellules du pancréas, appelées cellules β) participe également de manière importante, voire essentielle, au processus de lipogénèse évoqué ci-avant. En effet, l'une des étapes limitantes dans l'utilisation par les adipocytes du glucose extra-cellulaire contenu dans l'organisme en vue de sa tranformation finale en triglycérides réside dans la captation, le transport et la diffusion préalables de ce glucose à travers la membrane plasmique de la cellule adipocytaire seul le glucose ayant réussi à traverser cette membrane plasmique sera transformé en glycérophosphate puis en triglycérides. On sait aujourd'hui que ce transport du glucose est assuré par des glycoprotéines particulières et parfaitement identifiées, qui sont situées à la surface externe de la membrane plasmique, et que l'on appelle par simplicité "transporteurs-glucose" ou "GLUT". Or, il se trouve que certains de ces GLUT (dits transporteurs insulino-sensibles ou GLUT-4) sont en outre constitués de récepteurs protéiques spécifiques pour l'insuline sur lesquels (normalement) cette denière peut venir se fixer de façon très ferme (création de complexes protéiques du type insuline-récepteur de l'insuline). L'un des effets majeurs de l'insuline, en venant ainsi se lier sur ces récepteurs spécifiques, est de provoquer, selon des mécanismes d'activation complexes mais connus qu'il n'y a pas lieu de developper ici, un accroissement significatif du nombre de GLUT à la surface de l'adipocyte, générant par là une augmentation notable des quantités de glucose transportées à travers la membrane plasmique, et donc, par voie de conséquence, des quantités de triglycérides produites à l'intérieur de l'adipocyte. A cet égard, il est connu que des défauts prononcés, chez un organisme vivant, au niveau de la production de l'insuline ou encore au niveau du fonctionnement des récepteurs de cette dernière, sont responsables de diverses pathologies plus ou moins graves, et notamment d'une maladie appelée *diabète sucré*, dont il sera reparlé un peu plus en détails ci-dessous.

Si, pour des raisons diverses (nourriture trop riche, inactivité, vieillissement et autres), un déséquilibre métabolique substantiel s'installe dans l'organisme entre la synthèse des lipides (formation de triglycérides par réaction enzymatique entre des acides gras et le glycérophosphate provenant du glucose) et leur dégradation par lipolyse (décomposition enzymatique de triglycérides en acides gras et glycérol), c'est à dire plus précisément si les quantités de graisses formées par lipogénèse (les "entrées") deviennent notablement et constamment supérieures à celles qui sont éliminées par lipolyse (les "sorties"), il se produit alors dans les adipocytes une accumulation de triglycérides qui, si elle devient excessive, peut se traduire progressivement par l'apparition d'une peau épaisse, à surface souvent irrégulière ("peau d'orange") et de consistance plus ou moins flasque ou gélatineuse, donnant finalement à la silhouette un aspect général disgracieux pouvant évoluer entre la simple surcharge locale (lipodysmorphie), en passant par l'embonpoint certain, et enfin la réelle obésité.

L'obésité correspond à un état pathologique qui se caractérise par une hypertrophie généralisée et importante du tissu adipeux, liée à une augmentation excéssive à la fois du nombre, de la masse et du volume des adipocytes et ayant pour origine le déséquilibre métabolique indiqué ci-avant. Compte tenu du profond inconfort tant physique qu'esthétique, et parfois psychologique, qu'elle occasionne auprès des individus qui en sont atteints, l'obésité constitue de nos jours une affection de moins en moins bien supportée ou acceptée. De surcroit, et malheureusement, l'obésité s'accompagne également souvent de troubles métaboliques secondaires plus ou moins graves, et l'on sait ainsi en particulier que l'obésité prédispose fortement à l'apparition précoce de certains types de diabètes sucrés. Le diabète sucré, qui est aux Etats-Unis la sixième, dans l'ordre, des maladies mortelles, se définit, d'une manière générale, comme un état pathologique d'hyperglycémie sanguine, et, à ce jour, on distinque plus précisément deux type principaux de diabètes sucrés, selon l'origine de cet excès de glucose dans le sang : le diabète dit insulino-dépendant (ou DID), encore appelé diabète de type I, et le diabète non insulino-dépendant (ou DNID) appelé aussi diabète de type II. Comme indiqué précédemment, l'insuline est un régulateur naturel de la concentration sanguine du glucose. Un déficit ou un tarissement de la production d'insuline dans l'organisme conduit au diabète de type I ; les individus atteints d'une telle affection doivent subir régulièrement des injections complémentaires d'insuline. L'autre cas correspond à celui où l'insuline, bien que présente en quantités apparemment normales dans l'organisme, est inactive, ou insuffisamment active, à l'égard du glucose, et ceci en raison de certaines déficiences au niveau des fonctions qui devraient être exercées par les récepteurs de l'insuline ; l'état de l'organisme est alors qualifié d'insulino-résistant, et doit être traité par des agents antidiabétiques spécifiques.

Les thiazolidinediones et leurs dérivés, et en particulier celui de formule 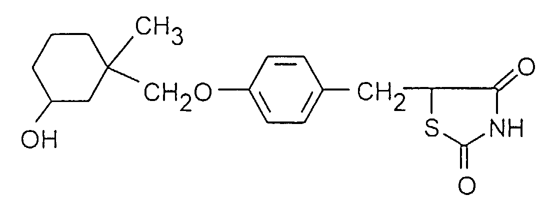 constituent des agents antidiabétiques particulièrement intéressants, reconnus comme permettant de corriger, *in vitro* et *in vivo*, l'état de résistance que peut présenter un organisme à l'action de l'insuline, et capables simultanément de stimuler le processus de différenciation des préadipocytes en adipocytes.

Toutefois, et malheureusement, l'un des inconvénients attachés à ce type de produits réside dans le fait que ceux-ci présentent, aux doses auxquelles il convient de les mettre habituellement en oeuvre, des effets secondaires indésirables plus ou moins importants.

### Exposé général de l'invention

Après d'importantes recherches menées sur la question, la Demanderesse vient maintenant de découvrir l'existence d'une synergie d'activité très importante entre certains composés pour activer et stimuler de manière particulièrement améliorée la différenciation des préadipocytes en adipocytes.

Plus précisément encore, il a été trouvé que l'association, nouvelle en soi, et notamment à titre de médicament, entre (i) une substance (ou ligand) présentant une affinité pour les récepteurs nucléaires de l'acide rétinoïque et/ou ses isomères (à savoir les recepteurs RARs et RXRs) et (ii) un acide gras, permettait de stimuler de manière tout à fait remarquable la différenciation préadipocytaire.

Il a également été trouvé par la Demanderesse que l'effet de synergie susmentionné était obtenu de manière optimale en mettant en oeuvre :
- de préférence un ligand spécifique des récepteurs RARs (par rapport aux récepteurs RXRs),
- encore plus préférentiellement, un ligand spécifique des RARs qui soit en outre sélectif à l'égard d'un ou deux (mais de préférence un seul) sous-types (RAR-α, RAR-β, RAR-γ) de ces RARs,
- et plus encore plus préférentiellement enfin, un ligand spécifique des RARs qui soit en outre sélectif à l'égard du récepteur RAR-α.

Toutes ces découvertes sont à la base de la présente invention.

Ainsi, dans l'un de ses premiers aspects, la présente invention a pour objet de nouvelles compositions, notamment médicamenteuses, qui sont essentiellement caractérisées par le fait qu'elles comprennent, dans un support physiologiquement acceptable et en particulier compatible avec le mode d'administration retenu, (i) au moins un ligand présentant une affinité pour les récepteurs nucléaires de l'acide rétinoïque et/ou ses isomères, de préférence un ligand spécifique des récepteurs RARs et encore plus préférentiellement un ligand sélectif pour le récepteur RAR-α, et (ii) au moins un acide gras. Ces deux composés peuvent être, dans la composition, associés physiquement (mélange) ou au contraire présents séparément dans des compartiments distincts (composition sous forme de "kits").

Selon un autre aspect de la présente invention, il est ainsi également proposé des dispositifs à plusieurs compartiments ou "kits" plus particulièrement destinés à la mise en oeuvre du procédé ci-dessous, et qui sont caractérisés par le fait qu'ils comprennent dans un premier compartiment un ou plusieurs ligands présentant une affinité pour les récepteurs nucléaires de l'acide rétinoïque et/ou ses isomères, de préférence un ligand spécifique des récepteurs RARs et encore plus préférentiellement un ligand sélectif pour le récepteur RAR-α, et, dans un deuxième compartiment, un ou plusieurs acides gras, les compositions contenues dans lesdits premier et second compartiments étant ici considérées comme compositions de combinaison pour une utilisation simultanée, séparée ou étalée dans le temps dans un procédé de traitement destiné à favoriser et/ou stimuler la différenciation des préadipocytes en adipocytes.

L'invention a également pour objet l'utilisation des compositions et/ou des kits ci-dessus à titre de, ou pour la fabrication de, compositions pharmaceutiques plus particulièrement destinées à stimuler la différenciation des préadipocytes en adipocytes, notamment pour le traitement des états d'insulino-résistance et/ou des physiopathologies associées.

L'invention a également enfin pour objet un procédé visant à stimuler la différenciation des préadipocytes en adipocytes notamment pour le traitement des états d'insulino-résistance et/ou des physiopathologies associées à de tels états, et qui est caractérisé par le fait qu'il consiste à administrer à l'organisme, de préférence par voie systémique, d'une part au moins un ligand présentant une affinité pour les récepteurs nucléaires de l'acide rétinoïque et/ou ses isomères, de préférence un ligand spécifique pour les récepteurs RARs et encore plus préférentiellement un ligand sélectif à l'égard du récepteur RAR-α, et d'autre part au moins un acide gras, l'apport de ces différents composés à l'organisme pouvant être réalisé de manière simultanée, séparée ou encore étalée dans le temps. De préférence, elle est effectuée de façon simultanée.

Le procédé selon l'invention peut bien entendu être appliqué directement sur des cultures cellulaires appropriées de préadipocytes (traitement *in vitro*).

D'une manière générale, les doses d'actifs qu'il convient de mettre en oeuvre, en association, pour obtenir l'effet technique premier recherché restent, selon l'invention, toujours très faibles (synergie), ce qui constitue un avantage important lorsqu'il s'agit de faire face à des problèmes de tolérance ou d'effets secondaires indésirables susceptibles d'apparaitre au sein des organismes à traiter ou en cours de traitement.

Les associations conformes à l'invention trouvent naturellement, compte tenu des activités remarquables qu'elles présentent vis à vis de la différenciation des cellules préadipocytaires, des applications privilégiées dans le traitement curatif et/ou prophylactique des patients touchés d'insulino-résistance ou de toutes autres physiopathologies associées à cet état. De telles physiopathologies sont notamment les diabètes de type II, ainsi que les maladies cardiovasculaires telles que par exemple l'hypertension et l'athérosclérose. L'état d'insulino-résistance chez un patient peut être détecté classiquement au moyen du test de tolérence au glucose, et le traitement selon l'invention peut être entrepris dès que ce test se révèle positif, avant même toute manifestation clinique d'un début de maladie (traitement préventif).

Mais d'autres caractéristiques, aspects, objets et avantages de l'invention apparaitront encore plus clairement à la lecture de la description et des figures qui vont suivre, ainsi que des divers exemple concrets, mais nullement limitatifs, destinés à l'illustrer.

### Exposé détaillé de l'invention

On commencera par décrire les différents actifs qui sont utilisés en association dans le cadre de la présente invention.

### Les Ligands (ou Rétinoïdes) spécifiques

On sait que l'acide rétinoïque, qui est un métabolite naturel de la Vitamine A (rétinol), est un puissant modulateur (i.e. un inhibiteur ou, au contraire, un stimulateur, selon la nature des cellules traitées) de la différenciation et de la prolifération de nombreux types cellulaires normaux ou transformés.

L'acide rétinoïque tout-*trans* (all-*trans* retinoic acid) agit sur la différenciation et la prolifération des cellules en interagissant avec des récepteurs nucléaires ou RARs (Retinoic Acid Receptors) contenus dans le noyau cellulaire. Il existe, à ce jour, trois sous-types identifiés de récepteurs RAR appellés respectivement RAR-α, RAR-β et RAR-γ. Ces récepteurs, après fixation du ligand (i.e. de l'acide rétinoïque), interagissent avec la région promotrice de gènes régulés par l'acide rétinoïque au niveau d'éléments de réponses spécifiques. Pour se fixer sur les éléments de réponse, les RARs s'hétérodimèrisent avec un autre type de récepteurs connus sous le nom de RXRs. Le ligand naturel des RXRs est l'acide 9-*cis*-rétinoïque.

De nombreux analogues structuraux synthétiques de l'acide rétinoïque ou de l'acide 9-*cis*-rétinoïque, couramment dénommés "rétinoïdes", ont par ailleurs été décrits à ce jour dans la littérature. Certaines de ces molécules sont capables de se fixer et d'activer spécifiquement les RARs ou, au contraire, les RXRs. De plus, certains analogues peuvent se fixer et activer un sous-type de récepteur RAR (α, β ou γ) particulier. D'autres analogues, enfin, ne présentent aucune affinité sélective particulière vis à vis de ces différents récepteurs. A cet égard, et par exemple, l'acide 9-*cis* rétinoïque active à la fois les RARs et les RXRs, sans sélectivité notable pour l'un ou l'autre de ces récepteurs (ligand non spécifique), alors que l'acide rétinoïque tout-*trans* active, quant à lui, spécifiquement les RARs (ligand spécifique RARs), tous sous-types confondus. Au sens de la présente invention, et qualitativement, une substance donnée (ou ligand) est dite sélective, ou spécifique, vis à vis d'une famille de récepteurs donnée (respectivement vis à vis d'un récepteur particulier de cette famille) lorsque ladite substance présente une forte, ou très forte, affinité pour l'ensemble des récepteurs de cette famille (respectivement pour le récepteur particulier de cette famille) et qu'elle présente par ailleurs une faible, ou très faible, affinité pour tous les récepteurs de toute autre famille (respectivement pour tous les autres récepteurs, de cette même famille ou pas). Quantitavement, l'affinité se mesure au moyen des techniques de binding classiques (valeurs Kd) et, selon la présente invention, toute substance qui, à l'égard d'un premier recepteur donné, présente un Kd au moins 5 fois inférieur, et de préférence au moins 10 fois inférieur, au Kd qu'elle présente à l'égard d'un deuxième récepteur donné, peut être qualifiée de substance selective vis à vis de ce premier récepteur par rapport à ce deuxième récepteur ; elle est qualifiée de spécifique lorsque le rapport entre lesdits Kd est d'au moins 100. L'évaluation du caractère sélectif ou non, ou spécifique ou non, d'une substance donnée à l'égard d'un récepteur donné est classiquement réalisée au moyen de tests *in vitro* bien connus de l'homme de l'art (voir notamment à cet égard : (1) "Selective Synthetic Ligands for Nuclear Retinoic Acid Receptor Subtypes" *in* RETINOIDS, Progress in Research and Clinical Applications, Chapitre 19 (pp 261-267), Marcel Dekker Inc, édité par Maria A. Livrea et Lester Packer ; (2) "Synthetic Retinoids : Receptor Selectivity and Biological Activity" *in* Pharmacol Skin, Basel, Karger, 1993, Volume. 5, pp 117-127 ; (3) "Selective Synthetic Ligands for Human Nuclear Retinoic Acid Receptors" *in* Skin Pharmacology, 1992, Vol. 5, pp 57-65 ; (4) "Identification of Synthetic Retinoids with Selectivity for Human Nuclear Retinoic Acid Receptor -γ" *in* Biochemical and Biophysical Research Communications, Vol. 186, N° 2, Juillet 1992, pp 977-983 ; (5) Demande de brevet internationale WO 93/21146).

Selon la présente invention, on ne retient, de préférence, des rétinoïdes (naturels ou synthétiques) ci-dessus que ceux qui présentent une affinité spécifique pour les récepteurs RARs, c'est à dire des ligands présentant une affinité plus grande pour ces derniers récepteurs que pour les récepteurs RXRs.

Selon un autre mode préféré de réalisation de la présente invention, les rétinoïdes spécifiques RARs sont choisis au sein de ceux qui présentent en outre une affinité sélective pour l'un au moins des récepteur RAR-α, RAR-β et RAR-γ, de préférence le récépteur RAR-α.

Il est bien entendu possible, selon la présente invention, de mettre en oeuvre un ou plusieurs ligands présentant une affinité pour les récepteurs nucléaires de l'acide rétinoïque et/ou ses isomères, ou un ou plusieurs ligands présentant une affinité spécifique vis à vis des récepteurs RARs, ou un ou plusieurs ligands présentant une affinité sélective vis à vis du récepteur RAR-α, ou encore des associations entre ces différents ligands.

A titre d'exemples de rétinoïdes à la fois spécifiques RARs et sélectifs RAR-α qui sont utilisables dans le cadre de la présente invention, on peut plus particulièrement citer l'acide 4-[(5,6,7,8-tétrahydro-5,5,8,8-tétraméthyl-2-naphtyl) carboxamido]benzoïque et l'acide 4-[(5,6,7,8-tétrahydro-5,5,8,8-tétraméthyl-2-naphtyl) carbamoyl]benzoïque.

De manière plus générale, comme exemples de rétinoïdes spécifiques des RARs, on peut notamment citer l'acide rétinoïque tout-*trans*, l'acide 4-[(5,6,7,8-tétrahydro-5,5,8,8-tétraméthyl-2-anthracényl]benzoïque, l'acide 4-[(5,6,7,8-tétrahydro-5,5,8,8-tétraméthyl-2-naphtalényl)carboxamido]benzoïque, l'acide 6-[3-(1-adamantyl)-4-hydroxyphényl]-2-naphtoïque, l'acide 6-[3-(1-méthylcyclohexyl)-4-méthoxyphényl]-2-naphtoïque, et l'adapalène.

### Les Acides Gras

Les acides gras utilisables dans le cadre de la présente invention peuvent être des acides gras saturés ou, au contraire, insaturés. Parmi ces derniers, on préfère mettre en oeuvre des acides gras polyinsaturés, et particulier les acides gras polyinsaturés en C₁₈-C₂₂ (dont les acides gras dits "essentiels") et notamment ceux en C₂₀. Il peut en outre s'agir d'acides gras métabolisables ou non métabolisables. On peut également mettre en oeuvre des précurseurs d'acides gras, c'est à dire des composés qui peuvent être métabolisés *in vivo* par l'organisme en acides gras ou bien encore des composés qui peuvent induire la formation d'acides gras polyinsaturés dans les tissus vivants, ceci pouvant être objectivé par chromatographie gazeuse ou par toute autre technique standard comme celles décrites par Pelick et al. P23 "Analysis of lipids and lipoproteins" éditions Perkins American Oil Chemist Society, Champaign Illinois U.S.A..

A titre d'exemples d'acides gras convenant particulièrement bien pour la mise en oeuvre de la présente invention, on peut notamment citer l'acide arachidonique, l'acide dihomogamma-linolénique, l'acide éicosapentaénoïque, l'acide docosahexaénoïque, les acides oléïque, linoléïque, α-linolénique et γ-linolénique, ainsi que l'acide palmitique et en particulier l'acide α-bromopalmitique.

Selon un mode préférentiel de réalisation de la présente invention, on met en oeuvre des acides gras choisis au sein des acides gras polyinsaturés de type ω₃ et ω₆. Parmi de tels acides gras, on peut plus particulièrement citer l'acide α-linolénique et l'acide arachidonique.

Les acides gras ci-dessus (ou leurs précurseurs) peuvent être obtenus notamment à partir de certains composés naturels, en particulier de certains aliments, d'origine animale, végétale ou microbienne (extraits d'huiles végétales telles que l'huile de Oénothéra biennis, l'huile de bourrache, l'huile de pépin de cassis, l'huile d'onagre, extraits d'huiles de poissons, extraits d'huiles de tissus d'insectes). Selon l'invention, il est bien entendu possible d'utiliser directement de tels composés naturels qui contiennent les acides gras ou les précurseurs d'acides gras désirés. Il est également possible d'utiliser des produits de synthèse. Enfin, on notera qu'il est bien évidemment tout à fait possible, selon la présente invention, d'utiliser des mélanges d'acides gras.

D'une manière générale, les rétinoïdes et les acides gras qui sont mis en oeuvre dans le cadre de la présente invention peuvent être classiquement conditionnés sous une forme convenant au mode d'administration ou d'application retenus finalement pour ces derniers. L'administration des compositions selon l'invention peut ainsi être effectuée par voie entérale, parentérale ou encore transcutanée (avec ou sans mise en oeuvre d'accélérateur(s) de pénétration). Toutefois, de préférence, ces dernières compositions sont conditionnées sous une forme convenant à une application par voie entérale.

Les compositions plus particulièrement visées par la présente invention sont donc des compositions de type pharmaceutiques contenant dans un support physiologiquement acceptable au moins un rétinoïde spécifique pour les récepteurs RARs, et qui soit de préférence en outre sélectif pour le récepteur RAR-α, à titre de premier principe actif, en association avec au moins un acide gras, présent à titre de second principe actif, lesdites compositions étant formulées et conditionnées de préférence sous une forme adaptée à une administration par voie systémique, et encore plus avantageusement orale. Les mêmes considérations s'appliquent au cas des "kits" conformes à l'invention ; en particulier, les compositions rentrant dans chacun des compartiments du kit sont de préférence formulées sous une forme convenant à une administration par voie systémique, avantageusement orale. A cet égard, on notera que selon la présente invention, il est en fait possible de concevoir des kits de présentation contenant autant de compartiments séparés que de substances actives (rétinoïdes, acides gras) que l'on désire ou qu'il est souhaitable de mettre en oeuvre.

Pour la voie entérale, les médicaments selon l'invention peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée.

Pour la voie parentérale, les médicaments peuvent se présenter sous forme de solutions ou de suspensions pour perfusion ou pour injection.

Les compositions selon l'invention, ou les kits selon l'invention, ou la mise en oeuvre du procédé selon l'invention, peuvent faire appel aux divers excipients et autres additifs classiques qui sont habituellement rencontrés dans le domaine de la pharmacopée (colorants, agents de texture, parfums, conservateurs et autres).

Les quantités de rétinoïde(s) spécifique(s) à mettre en oeuvre dans le cadre de la présente invention ne sont pas véritablement critiques et peuvent ainsi varier dans d'assez larges limites. Dans le cas de préparations destinées à une administration par voie systémique, les doses doivent rester compatibles avec les impératifs classiques liés à la toxicologie et la galénique des produits pharmaceutiques; à cet égard, des doses d'administration comprises entre 0,005 mg/Kg.jour et 5 mg/Kg. jour conviennent généralement.

De même, les quantités d'acide(s) gras à administrer sont généralement comprises entre 1 mg/Kg.jour et 50 mg/Kg. jour

Pour obtenir des effets notables, les fréquences d'administration des compositions selon l'invention, qui sont bien entendu fonctions des quantités d'agents actifs mises en oeuvre à chaque opération, sont de l'ordre de une à deux fois par jour. Le traitement est ensuite poursuivi régulièrement, pendant plusieurs jours, de préférence pendant plusieurs semaines, voire plusieurs mois.

On va maintenant donner, à titre nullement limitatif, plusieurs exemples destinés d'une part à démontrer les effets attachés à la présente invention, et, d'autre part, à illustrer diverses formulations concrètes conformes à l'invention.

### EXEMPLE 1

Cet exemple a pour but de mettre en évidence l'activité in vitro des associations synergétiques conformes à l'invention sur la différentiation de cellules préadipocytaires en cellules adipocytaires.

Les substances testées étaient les suivantes :
- rétinoïdes :
   R1 : acide 4-[(5,6,7,8-tétrahydro-5,5,8,8-tétraméthyl-2-anthracényl] benzoïque (rétinoïde spécifique RARs, sans sélectivité particulière sur les sous-types α, β ou γ))
   R2 : acide 4-[(5,6,7,8-tétrahydro-5,5,8,8-tétraméthyl-2-naphtalényl) carboxamido]benzoïque (rétinoïde spécifique RARs et sélectif sur RAR-α)
   R3 : acide 6-[3-(1-méthylcyclohexyl)-4-méthoxyphényl]-2-naphtoïque (rétinoïde spécifique RARs et sélectif RAR-β)
   R4: l'acide 6-[3-(1-adamantyl)-4-hydroxyphényl]-2-naphtoïque (rétinoïde spécifique RARs et sélectif RAR-γ)
   R5 : acide 9-cis rétinoïque (rétinoïde non spécifique)
- acide gras : acide α-bromopalmitique

Les cellules préadipocytaires utilisées sont des cellules issues de la lignée Ob1771.

Le protocole expérimental et les méthodes de détermination des activités en différentiation, étaient les suivants :

### Culture des cellules:

Les cellules sont cultivées à 37°C dans une atmosphère humide en présence de 5% de CO₂ dans un milieu DME (Gibco) additionné d'antibiotiques (penicilline à 100 U/ml et streptomycine à 50 mg/ml) et de 8% (v/v) de serum de veau fétal (SVF)(milieu standart).

### Traitement des cellules:

Les cellules Ob1771 sont ensemencées à raison de 2.10⁴ cellules dans 2 ml du milieu standart additionné de 17 nM d'insuline, 2 nM de triiodothyronine, 100 µM de putrescine et 10 µM de méthylglyoxal bis(guanyl-hydrazone) dans des puits de cluster de 10 cm². Les cellules sont traitées dès l'ensemencement avec les rétinoïdes définis ci-dessus et/ou l'acide α-bromopalmitique dilués dans de l'éthanol. La concentration finale en éthanol n'excède pas 0,1% (v/v). Après 8 jours de culture, les cellules sont récoltées pour la détermination de la différenciation.

### Mesure de la différenciation:

La différenciation est déterminée au moyen de l'activité glycérophosphate déshydrogénase (GPDH) mesurée spectrophotométriquement à 340 nm. Elle est exprimée en mU (µmol/min)/mg de protéine. Plus l'activité est forte, plus la proportion de cellules différenciées est élevée. Les résultats sont exprimés par rapport à l'activité déterminée dans les cellules non exposées aux rétinoïdes et/ou l'acide gras (35 ± 5 mU/mg).

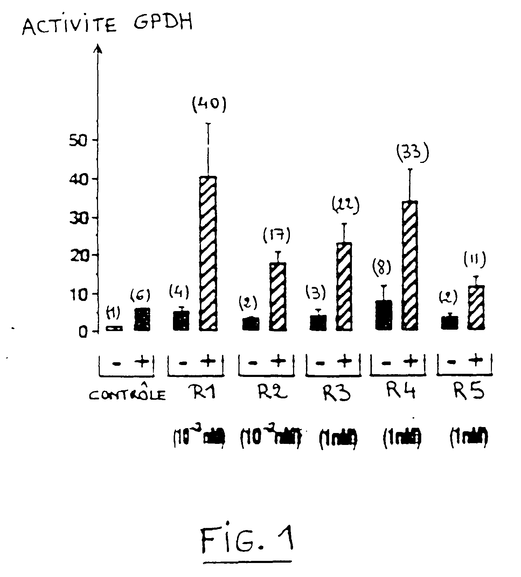

Tous les résultats obtenus sont rassemblés dans la figure 1. Cette figure quantifie l'évolution du taux de préadipocytes différenciées en fonction des actifs utilisés d'une part et de leurs concentrations d'autre part (R1 : 10⁻³ nM ; R2 : 10⁻² nM ; R3 : 1 nM ; R4 : 1 nM ; R5 : 1 nM). Dans tous les cas, la concentration en acide gras (lorsque présent) était fixée à 5 µM; (+) indique la présence de cet acide gras et (-) son absence. Les valeurs données au point de contrôle correspondent aux résultats trouvés lorsqu'on ne met en oeuvre aucun rétinoïde (dans ce dernier cas, (-) correspond aux valeurs trouvées lorsqu'on ne met en oeuvre aucun actif (absence d'acide gras) et (+) à celles trouvées en ne mettant en oeuvre que l'acide gras à la concentration de 5 µM).

Ces résultats mettent clairement en évidence les effets de synergie obtenus dans le cadre des associations conformes à l'invention, la quantité de cellules différenciées étant en effet systématiquement supérieure à la simple somme arithmétique des quantités de cellules différenciées obtenues lorsqu'on met en oeuvre séparément et isolément les rétinoïdes et l'acide gras.

### EXEMPLE 2

On illustre ici trois formulations concrètes conformes à l'invention et se présentant sous une forme convenant à une administration par voie orale.
(a) Capsule de 1 g contenant 0,5 g d'une suspension huileuse constituée de :
   - Composé R1 de l'exemple 1 0,25 mg
   - Acide α-linoléique 250 mg
   - Huile de paraffine qsp 500 mg

   L'enveloppe de la capsule est fabriquée selon une technique classique de moulage puis séchage d'un mélange approprié comprenant : gélatine, glycérine, eau et conservateur. Les opérations de mélange puis de soutirage des ingrédients formant la suspension huileuse sont effectuées sous gaz inerte.
(b) Gélule (enveloppe standard calibrée M°3 opaque) de 0,30 ml contenant :
   - Adapalène 5 mg
   - Acide α-bromopalmitique 50 mg
   - Silice ("AEROSIL 200" de chez Degussa) 50 mg
   - Lactose qsp 0,3 ml
(c) Comprimé non soluble de 0,5 g constitué de :
   - Composé R2 de l'exemple 1 2,5 mg
   - Acide α-bromopalmitique 100 mg
   - Lactose 85 mg
   - Talc purifié 15 mg
   - Edulcorant qs
   - Colorant qs
   - Amidon qsp 500 mg

## Revendications

1. Composition, caractérisée par le fait qu'elle comprend, dans un support physiologiquement acceptable, (i) au moins un ligand présentant une affinité pour les récepteurs nucléaires de l'acide rétinoïque et/ou de ses isomères et (ii) au moins un acide gras.

2. Composition selon la revendication 1, caractérisée par le fait que ledit ligand est un ligand qui présente une affinité spécifique pour les récepteurs RARs.

3. Composition selon la revendication 2, caractérisée par le fait que ledit ligand spécifique RARs ne présente pas d'affinité sélective pour l'un des sous-types de récepteurs RARs.

4. Composition selon la revendication 2, caractérisée par le fait que ledit ligand spécifique RARs présente en outre une affinité sélective à l'égard de l'un au moins des sous-types de récepteurs RARs.

5. Composition selon la revendication 4, caractérisée par le fait que ledit sous-type est le récepteur RAR-α.

6. Composition selon l'une quelconque des revendications précédentes, caractérisée par le fait que ledit ligand est choisi parmi l'acide 4-[(5,6,7,8-tétrahydro-5,5,8,8-tétraméthyl-2-naphtyl)carboxamido]benzoïque, l'acide 4-[(5,6,7,8-tétrahydro-5,5,8,8-tétraméthyl-2-naphtyl)carbamoyl]benzoïque, l'acide rétinoïque tout-*trans*, l'acide 4-[(5,6,7,8-tétrahydro-5,5,8,8-tétraméthyl-2-anthracényl]benzoïque, l'acide 4-[(5,6,7,8-tétrahydro-5,5,8,8-tétraméthyl-2-naphtalényl)carboxamido]benzoïque, l'acide 6-[3-(1-adamantyl)-4-hydroxyphényl]-2-naphtoïque, l'acide 6-[3-(1-méthylcyclohexyl)-4-méthoxyphényl]-2-naphtoïque, et l'adapalène.

7. Composition selon l'une quelconque des revendications précédentes, caractérisée par le fait que ledit acide gras est un acide gras saturé ou insaturé.

8. Composition selon la revendication 7, caractérisé par le fait que ledit acide gras est un acide gras polyinsaturé.

9. Composition selon la revendication 8, caractérisée par le fait que ledit acide gras polyinsaturé contient de 18 à 22 atomes de carbone.

10. Composition selon la revendication 8 ou 9, caractérisée par le fait que ledit acide gras polyinsaturé est du type ω₃ ou ω₆.

11. Composition selon l'une quelconque des revendications précédentes, caractérisée par le fait que ledit acide gras est choisi parmi l'acide arachidonique, l'acide dihomogamma-linolénique, l'acide éicosapentaénoïque, l'acide docosahexaénoïque, l'acide oléïque, l'acide linoléïque, l'acide α-linolénique, l'acide γ-linolénique, l'acide palmitique et l'acide α-bromopalmitique.

12. Composition selon l'une quelconque des revendications précédentes, caractérisée par le fait qu'il s'agit d'une composition conditionnée sous une forme convenant à un usage systémique, de préférence entéral.

13. Composition selon l'une quelconque des revendications précédentes, caractérisée par le fait qu'elle est conditionnée sous la forme d'un dispositif à plusieurs compartiments ou "kit", dont l'un des compartiments contient ledit ligand et un autre compatiment ledit acide gras.

14. Composition selon l'une quelconque des revendications précédentes, pour une utilisation comme médicament.

15. Composition selon la revendication 14, pour une utilisation comme stimulateur de la différenciation des préadipocytes en adipocytes.

16. Composition selon l'une des revendications 14 ou 15, pour une utilisation destinée au traitement d'un état d'insulino-résistance.

17. Composition selon l'une des revendications 14 à 16, pour une utilisation destinée au traitement d'une physiopathologie associée à un état d'insulino-résitance.

18. Composition selon la revendication 17, pour une utilisation destinée au traitement des diabètes de type II, et des maladies cardiovasculaires telles que l'hypertension et l'athérosclérose.

19. Utilisation d'une composition telle que définie à l'une quelconque des revendications 1 à 13 pour la fabrication d'une composition pharmaceutique destinée à stimuler la différenciation des préadipocytes en adipocytes et/ou à traiter l'une des affections définies aux revendications 16 à 18.

## Patentansprüche

1. Zusammensetzung, dadurch gekennzeichnet, daß sie in einem physiologisch akzeptablen Träger (i) mindestens einen Liganden, der eine Affinität für die Kernrezeptoren der Retinoesäure und/oder ihrer Isomere aufweist, und (ii) mindestens eine Fettsäure enthält.

2. Zusammensetzung nach Anspruch 1, dadurch gekennzeichnet, daß der Ligand ein Ligand ist, der eine spezifische Affinität für die RAR-Rezeptoren aufweist.

3. Zusammensetzung nach Anspruch 2, dadurch gekennzeichnet, daß der für die RAR-Rezeptoren spezifische Ligand keine selektive Affinität für einen der Subtypen der RAR-Rezeptoren aufweist.

4. Zusammensetzung nach Anspruch 2, dadurch gekennzeichnet, daß der für die RAR-Rezeptoren spezifische Ligand außerdem eine selektive Affinität für mindestens einen der Subtypen der RAR-Rezeptoren aufweist.

5. Zusammensetzung nach Anspruch 4, dadurch gekennzeichnet, daß es sich bei dem Subtyp um den RAR-α-Rezeptor handelt.

6. Zusammensetzung nach einem der vorhergehenden Ansprüche, dadurch gekennzeichnet, daß der Ligand ausgewählt ist unter 4-[(5,6,7,8-Tetrahydro-5,5,8,8-tetramethyl-2-naphtyl)-carboxamido]-benzoesäure, 4-[(5,6,7,8-Tetahydro-5,5,8,8-tetramethyl-2-naphthyl)-carbamoyl]-benzoesäure, all-*trans*-Retinoesäure, 4-[(5,6,7,8-Tetrahydro-5,5,8,8-tetramethyl-2-anthracenyl]-benzoesäure, 4-[(5,6,7,8-Tetahydro-5,5,8,8-tetramethyl-2-naphtalenyl)-carboxamido]-benzoesäure, 6-[3-(1-Adamantyl)-4-hydroxyphenyl]-2-naphthoesäure, 6-[3-(1-Methylcyclohexyl)-4-methoxyphenyl]-2-naphthoesäure und Adapalen.

7. Zusammensetzung nach einem der vorhergehenden Ansprüche, dadurch gekennzeichnet, daß die Fettsäure eine gesättigte oder ungesättigte Fettsäure ist.

8. Zusammensetzung nach Anspruch 7, dadurch gekennzeichnet, daß die Fettsäure eine mehrfach ungesättigte Fettsäure ist.

9. Zusammensetzung nach Anspruch 8, dadurch gekennzeichnet, daß die mehrfach ungesättigte Fettsäure 18 bis 22 Kohlenstoffatome enthält.

10. Zusammensetzung nach Anspruch 8 oder 9, dadurch gekennzeichnet, daß die mehrfach ungesättigte Fettsäure eine Fettsäure vom ω₃- oder ω₆-Typ ist.

11. Zusammensetzung nach einem der vorhergehenden Ansprüche, dadurch gekennzeichnet, daß die Fettsäure unter Arachidonsäure, Dihomo-γ-linolensäure, Eicosapentaensäure, Docosahexaensäure, Ölsäure, Linolsäure, α-Linolensäure, γ-Linolensäure, Palmitinsäure und α-Brompalmitinsäure ausgewählt ist.

12. Zusammensetzung nach einem der vorhergehenden Ansprüche, dadurch gekennzeichnet, daß es sich um eine Zusammensetzung handelt, die in einer Form verpackt ist, die für eine systemische, vorzugsweise enterale Verabreichung geeignet ist.

13. Zusammensetzung nach einem der vorhergehenden Ansprüche, dadurch gekennzeichnet, daß sie in Form einer Einrichtung mit mehreren Abteilungen oder "Kit" verpackt ist, von denen eine Abteilung den Liganden und eine andere Abteilung die Fettsäure enthält.

14. Zusammensetzung nach einem der vorhergehenden Ansprüche zur Verwendung als Arzneimittel.

15. Zusammensetzung nach Anspruch 14 zur Verwendung für die Stimulierung der Differenzierung von Präadipocyten zu Adipocyten.

16. Zusammensetzung nach einem der Ansprüche 14 und 15 für eine Verwendung, die für die Behandlung eines Insulinresistenz-Zustands vorgesehen ist.

17. Zusammensetzung nach einem der Ansprüche 14 bis 16 für eine Verwendung, die für die Behandlung einer physiologischen Erkrankung vorgesehen ist, die mit einem Insulinresistenz-Zustand verbunden ist.

18. Zusammensetzung nach Anspruch 17 für eine Verwendung, die für die Behandlung des Typ-II-Diabetes und von Herz-Kreislauf-Erkrankungen, wie Bluthochdruck und Arteriosklerose, vorgesehen ist.

19. Verwendung einer wie in einem der Ansprüche 1 bis 13 definierten Zusammensetzung für die Herstellung einer pharmazeutischen Zusammensetzung, die für die Stimulierung der Differenzierung von Präadipocyten zu Adipocyten und/oder die Behandlung einer der in den Ansprüchen 16 bis 18 definierten Erkrankungen vorgesehen ist.

## Claims

1. Composition, characterized in that it comprises, in a physiologically acceptable support, (i) at least one ligand with affinity for the nuclear receptors of retinoic acid and/or its isomers, and (ii) at least one fatty acid.

2. Composition according to Claim 1, characterized in that the said ligand is a ligand which has specific affinity for the RAR receptors.

3. Composition according to Claim 2, characterized in that the said RAR-specific ligand has no selective affinity for one of the RAR-receptor subtypes.

4. Composition according to Claim 2, characterized in that the said RAR-specific ligand also has selective affinity towards at least one of the RAR-receptor subtypes.

5. Composition according to Claim 4, characterized in that the said subtype is the RAR-a receptor.

6. Composition according to any one of the preceding claims, characterized in that the said ligand is chosen from 4-[(5,6,7,8-tetrahydro-5,5,8,8-tetramethyl-2-naphthyl)carboxamido]benzoic acid, 4-[(5,6,7,8-tetrahydro-5,5,8,8-tetramethyl-2-naphthyl)carbamoyl]benzoic acid, *all-trans* retinoic acid, 4-[(5,6,7,8-tetrahydro-5,5,8,8-tetramethyl-2-anthracenyl)]benzoic acid, 4-[(5,6,7,8-tetrahydro-5,5,8,8-tetramethyl-2-naphthalenyl)carboxamido]benzoic acid, 6-[3-(1-adamantyl)-4-hydroxyphenyl]-2-naphthoic acid, 6-[3-(1-methylcyclohexyl)-4-methoxyphenyl]-2-naphthoic acid and adapalene.

7. Composition according to any one of the preceding claims, characterized in that the said fatty acid is a saturated or unsaturated fatty acid.

8. Composition according to Claim 7, characterized in that the said fatty acid is a polyunsaturated fatty acid.

9. Composition according to Claim 8, characterized in that the said polyunsaturated fatty acid contains from 18 to 22 carbon atoms.

10. Composition according to Claim 8 or 9, characterized in that the said polyunsaturated fatty acid is of the ω₃ or ω₆ type.

11. Composition according to any one of the preceding claims, characterized in that the said fatty acid is chosen from arachidonic acid, dihomo-gamma-linolenic acid, eicosapentaenoic acid, docosahexaenoic acid, oleic acid, linoleic acid, α-linolenic acid, γ-linolenic acid, palmitic acid and α-bromopalmitic acid.

12. Composition according to any one of the preceding claims, characterized in that it is a composition packaged in a form which is suitable for systemic use, preferably enteral use.

13. Composition according to any one of the preceding claims, characterized in that it is packaged in the form of a multicompartment device or "kit", one of the compartments of which contains the said ligand and another compartment of which contains the said fatty acid.

14. Composition according to any one of the preceding claims, for use as a medicinal product.

15. Composition according to Claim 14, for use as a stimulator of the differentiation of preadipocytes into adipocytes.

16. Composition according to either of Claims 14 and 15, for use intended for the treatment of an insulin-resistance condition.

17. Composition according to one of Claims 14 to 16, for use intended for the treatment of a physiopathology associated with an insulin-resistance condition.

18. Composition according to Claim 17, for use intended for the treatment of type-II diabetes, and cardiovascular diseases such as hypertension and atherosclerosis.

19. Use of a composition as defined in any one of Claims 1 to 13, for the manufacture of a pharmaceutical composition intended for stimulating the differentiation of preadipocytes into adipocytes and/or for treating one of the complaints defined in Claims 16 to 18.
